# EUROPÄISCHE PATENTSCHRIFT

(11) **EP 1 465 977 B1**
(45) Veröffentlichungstag und Bekanntmachung des Hinweises auf die Patenterteilung: **29.08.2007**
(21) Anmeldenummer: 03704378.3
(22) Anmeldetag: 11.01.2003
(51) Int. Cl.: C12M 1/22, C12M 3/06

(54) **VORRICHTUNG ZUM Z CHTEN ODER KULTIVIEREN VON ZELLEN IN EINEM DOSENARTIGEN BEH LTER**
DEVICE FOR RAISING OR CULTIVATING CELLS IN A CONTAINER-LIKE RECEPTACLE
DISPOSITIF DE CULTURE OU DE MISE EN CULTURE DE CELLULES DANS UN RECIPIENT DE TYPE BOITE

(30) Priorität: 15.01.2002 DE 10201259
(43) Veröffentlichungstag der Anmeldung: 13.10.2004
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen
(86) Internationale Anmeldenummer: PCT/EP2003/000211
(87) Internationale Veröffentlichungsnummer: WO 2003/060055

(56) Entgegenhaltungen:
- WO-A-02/24861
- DE-A- 19 631 997
- DE-A- 19 710 650
- GB-A- 2 305 936
- US-A- 2 701 229
- US-A- 5 219 755
- US-A- 5 267 791
- PATENT ABSTRACTS OF JAPAN vol. 1995, no. 03, 28. April 1995 (1995-04-28) -& JP 06 343457 A (POWER FUIIRUDO:KK), 20. Dezember 1994 (1994-12-20)
- PATENT ABSTRACTS OF JAPAN vol. 1996, no. 10, 31. Oktober 1996 (1996-10-31) -& JP 08 154663 A (ABLE KK), 18. Juni 1996 (1996-06-18)
- PATENT ABSTRACTS OF JAPAN vol. 2000, no. 06, 22. September 2000 (2000-09-22) & JP 2000 078963 A (KOIZUMI YOSHIO), 21. März 2000 (2000-03-21)

## Beschreibung

Die Erfindung betrifft eine Vorrichtung zum züchten oder Kultivieren von Zellen in einem dosenartigen Behälter, der einen Boden und wenigstens einen Deckel aufweist.

Für den Laborbedarf ist es bekannt, Zellkulturen in einem flachen Behälter bzw. einer Schale zu züchten oder zu kultivieren, wobei die Zellen einfach in den Behälter eingelegt und Nährmedium zugegeben wird. Anschließend wird auf den Behälter ein Deckel aufgelegt.

Nachteilig dabei ist, daß dieses Verfahren nur für geringe Mengen angewendet werden kann. Insbesondere für eine Serienkultivierung oder Serienzüchtung von Zellen ist das bekannte System nicht geeignet. Darüber hinaus können keine "in-vivo"-Verhältnisse erreicht werden und es ist keine Sterilität gesichert.

Eine Alternative dazu bestand darin, daß mit einem geschlossenen System gearbeitet wurde, das einen Behälter mit einem Deckel oder Verschluß aufwies, wobei eine Sterilität möglich war. Nachteilig dabei war jedoch, daß die Entnahme der Zellkultur sehr umständlich und aufwendig war.

Der vorliegenden Erfindung liegt daher die Aufgabe zugrunde, eine Vorrichtung der eingangs erwähnten Art derart zu verbessern, daß sie auf die vielfältigste Weise zum Züchten oder Kultivieren von Zellen verwendet werden kann, insbesondere im Massenbetrieb, wobei soweit wie möglich "in-vivo"-Verhältnisse und eine Sterilität erreicht werden sollen. Au-ßerdem sollen sich die Zellkulturen nach ihrer Züchtung auch ohne deren Beschädigung ohne großen Aufwand aus dem Behälter entnehmen lassen.

Erfindungsgemäß wird diese Aufgabe durch die im kennzeichnenden Teil von Anspruch 1 genannten Merkmale gelöst.

Entsprechend der erfindungsgemäßen Vorrichtung werden die Zellen in dem Behälter nunmehr "nicht mehr nur sich selbst überlassen", sondern es findet praktisch ein aktiver Prozeß statt. Zum einen kann kontinuierlich oder diskontinuierlich Nährmedium zugeführt werden und zum anderen ist es möglich, die sich bildenden Zellkulturen mit Druck zu beaufschlagen. Dabei kann die Druckbeaufschlagung sogar mit wechselndem Druck erfolgen, um natürliche Verhältnisse so weit wie möglich nachzubilden.

Die Zellen können dabei als separate Zellkulturen gezüchtet oder kultiviert werden. In gleicher Weise können sie auch auf Strukturen gebildet werden, um Implantate zu erzeugen.

In dem erfindungsgemäßen Behälter können auf diese Weise durch eine entsprechende Druckbeaufschlagung die Zellen auch Scher- oder Druckkräften ausgesetzt werden. Mit der erfindungsgemäßen Vorrichtung lassen sich die verschiedensten Zellkulturen auf sehr universelle Weise züchten oder kultivieren.

Der Behälter selbst ist für diesen Zweck mit zwei Deckeln versehen. Ebenso ist eine Anschlußbohrung für den Zulauf von Nährmedium und eine Anschlußbohrung für dessen Rücklauf vorgesehen.

Durch die erfindungsgemäße Ausgestaltung mit zwei Deckeln oder Deckel und Boden nebst dazwischenliegendem Behälter, der in einfacher Weise lediglich ein auf der Unterseite und der Oberseite offener Zylinder sein kann, können die Zellkulturen nach ihrer Behandlung bzw. Züchtung ohne großen Aufwand und ohne deren Schädigung auf einfache Weise aus dem Behälter entnommen werden.

Da erfindungsgemäß die Züchtung oder Kultivierung der Zellen auf dem unteren Deckel oder Boden oder auch unter dem oberen Deckel, z.B. auf einem mit dem oberen Deckel verbundenen Gestell erfolgt, können die Zellkulturen nach ihrer Fertigstellung auf einfache Weise aus dem Behälter entnommen werden. Auf jeden Fall ist durch die abnehmbaren Deckel oder Boden eine hohe Zugänglichkeit gegeben.

Für eine Druckbeaufschlagung kann der Behälter, z.B. die zylinderförmige Behälterumfangswand, auch elastisch ausgebildet sein.

Die Anschlußbohrungen können dabei in beiden Deckeln angeordnet sein. Ebenso ist es auch möglich, die Anschlußbohrungen in den zylinderförmigen Mittelteil einzubringen. Die Anzahl und die Anordnung der Anschlußbohrungen richtet sich dabei jeweils nach dem Einsatzfall und der zu züchtenden oder kultivierenden Zellen.

Für eine dichte Verbindung zwischen den Deckeln und dem Behälter sind erfindungsgemäß Gewindeverbindungen mit Innen- und Außengewinden vorgesehen.

Sehr gute Abdicht- und damit Druckverhältnisse ergeben sich, wenn der oder die Deckel mit Verlängerungsringen versehen sind, die dann außenseitig dichtend den zylinderförmigen Mittelteil des Behälters umschließen.

Wenn man die erfindungsgemäße Vorrichtung einer Roll- oder Drehbewegung unterwerfen möchte, können seitlich auf die Vorrichtung Spannringe aufgebracht werden, die den oder die Deckel und den Behälter umfassen und an denen dann Dreh- oder Rolleinrichtungen angebracht werden können.

Für ein Unterdrucksetzen des einen Zellkulturraum bildenden Inneren des Behälters sind die verschiedenartigsten Druckbeaufschlagungseinrichtungen möglich. Hierzu sind z.B. Zylinder-/Kolbeneinheiten geeignet, die auch für eine wechselnde Druckbelastung entsprechend pulsierend operieren können.

Im Bedarfsfalle kann man den Behälter auch als Zweikammersystem ausbilden, um zwei verschiedene Zellen oder auch zwei gleiche Zellen getrennt voneinander kultivieren oder züchten zu können.

In diesem Falle ist es von Vorteil, wenn man den Deckel des Behälters mit einer Hängeeinrichtung versieht, an der eine Plattform zur Aufnahme von Zellen angeordnet ist. Damit erfolgt die Züchtung von einer Zellenart auf der Plattform, während eine weitere Zellkultivierung auf dem Boden des Behälters erfolgen kann.

Der Behälter bzw. die zylindrische Umfangswand des Behälters kann im Bedarfsfalle ebenfalls porös bzw. gasdurchlässig ausgebildet sein, so daß auf diese Weise ebenfalls noch Nährmittel und/oder ein gasförmiges Medium, wie z.B. Luft oder Sauerstoff, von dieser Seite aus zugeführt werden kann.

Vorteilhafte Weiterbildungen und Ausgestaltungen ergeben sich aus den übrigen Unteransprüchen.

Es zeigt:
- Fig. 1: einen Schnitt durch eine Vorrichtung mit einem zylinderförmigen Mittelteil und einem oberen und einem unteren Deckel;
- Fig. 2: eine ähnliche Ausgestaltung wie in der Fig. 2 dargestellt, wobei ein Zulaufanschluß und ein Rücklaufanschluß im oberen Deckel angeordnet sind;
- Fig. 3: eine weitere Ausführungsform der Vorrichtungsform;
- Fig. 4: eine Vorrichtung mit einem oberen Deckel und einem unteren Deckel, jeweils mit Außengewinde und einem zylinderringförmigen Mittelteil mit Innengewinde;
- Fig. 5: eine Ausführungsform mit Verlängerungsringen an einem oberen Deckel und einem unteren Deckel;
- Fig. 6: eine ähnliche Ausgestaltung wie in Fig. 5 mit Gewindeverbindungen;
- Fig. 7: eine Ausführungsform mit zwei seitlichen Spannringen;
- Fig. 8: eine erfindungsgemäße Vorrichtung ähnlich der Ausführungsform nach Fig. 7 mit einer Druckbeaufschlagungseinrichtung;
- Fig. 9: eine Ausführungsform mit einer Hängeeinrichtung im oberen Deckel, mit einem zylinderförmigen Mittelteil und einem unteren Deckel;
- Fig. 10: eine ähnliche Ausgestaltung wie Fig. 9 in etwas einfacher Form mit nur einem oberen Deckel;

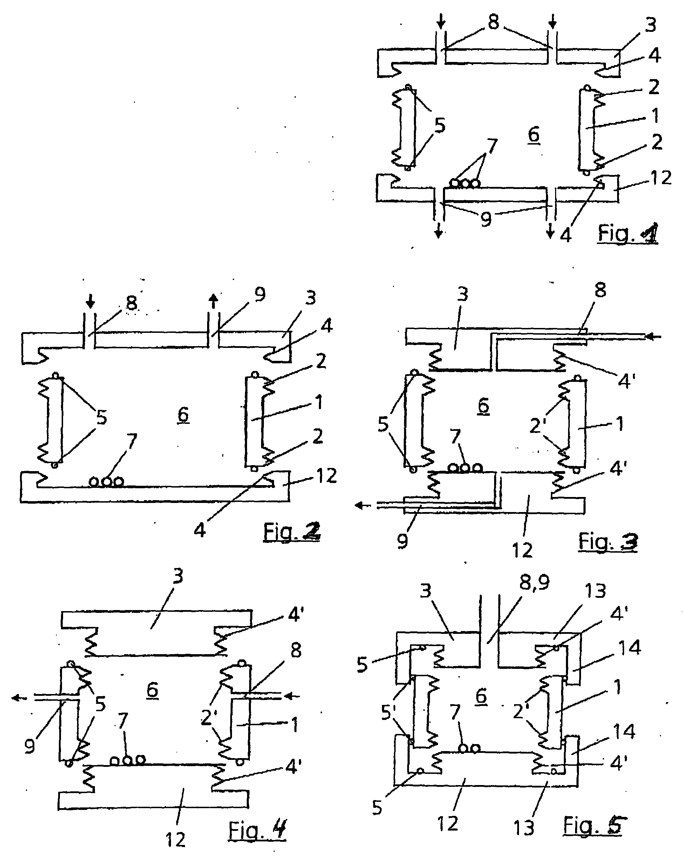

Fig. 1 zeigt eine Ausgestaltung, wobei der Behälter ein zylinderförmiges Mittelteil (1) bildet, der mit dem oberen Deckel 3 abgeschlossen werden kann. Auf der Unterseite ist ein weiterer unterer Deckel 12 vorgesehen, der den Boden des Behälters bildet und der ebenfalls ein Innengewinde 4 aufweist, welches mit dem Außengewinde 2 des Mittelteiles zusammenwirkt, wobei in diesem Fall das Mittelteil mit zwei Außengewinden 2 versehen ist, sofern nicht ein durchgehendes Gewinde vorgesehen ist. Wie weiterhin aus der Fig. 1 ersichtlich ist, sind dabei Zu- und Rücklaufanschlußbohrungen 8 und 9 in beiden Deckeln 3 und 12 vorgesehen.

Fig. 2 zeigt eine ähnliche Ausgestaltung wie Fig. 1, wobei eine Zulaufanschlußbohrung 8 und ein Rücklaufanschlußbohrung 9 nur im oberen Deckel 3 angeordnet sind. Ebenso wie bei dem Ausführungsbeispiel nach Fig. 2 sind an beiden Stirnseiten des zylinderringförmigen Mittelteiles (1) des Behälters Dichtringe 5 vorgesehen.

Fig. 3 zeigt eine Ausführungsform, wobei das zylinderförmige Mittelteil als Behälter 1 mit einem Innengewinde 2' versehen ist, welches jeweils mit einem Außengewinde 4' des oberen Deckels 3 und des unteren Deckels 12 zusammenarbeitet. Auch hier sind wiederum auf beiden Stirnseiten des Behälters Dichtringe 5 vorgesehen. In diesem Fall befindet sich eine Zulaufanschlußbohrung 8 im oberen Deckel 3 und eine Rücklaufanschlußbohrung 9 im unteren Deckel 12.

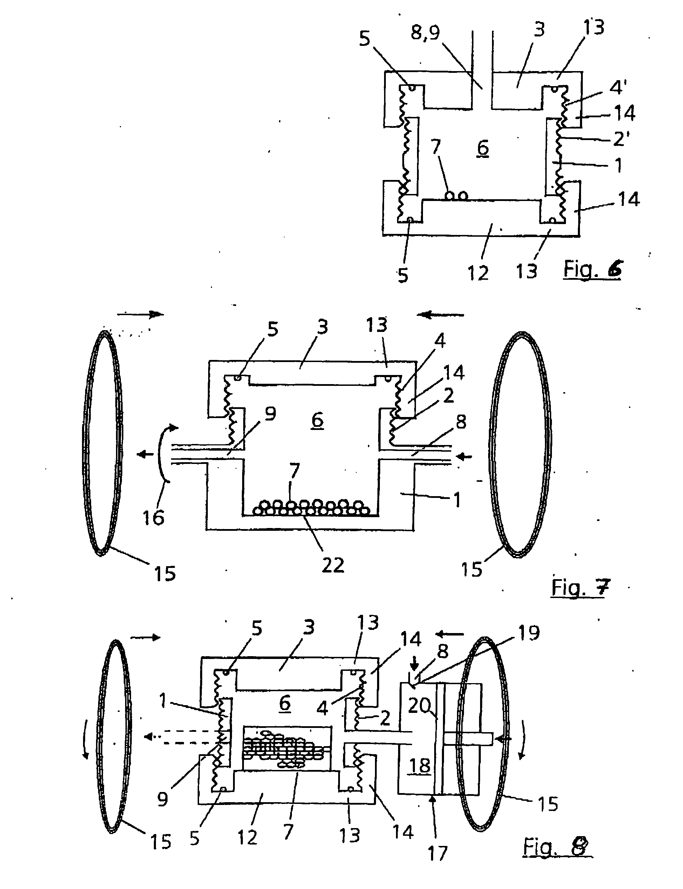

Fig. 4 zeigt eine ähnliche Ausgestaltung wie Fig. 3. Der wesentliche Unterschied besteht lediglich darin, daß die Zulaufanschlußbohrung 8 und die Rücklaufanschlußbohrung 9 sich gegenüberliegend in dem zylinderringförmigen Mittelteil (1) des Behälters angeordnet sind.

Fig. 5 zeig eine Ausführungsform mit einem zylinderringförmigen Mittelteil (1) als Behälter, einem oberen Deckel 3 und einem unteren Deckel 12. Dabei weisen beide Deckel 3 und 12 jeweils ein Außengewinde 4' auf, welches mit Innengewinden 2' des Behälters zusammenwirkt. Zusätzlich sind beide Deckel 3 und 12 mit radialen Erweiterungen 13 versehen, von deren äußeren Enden aus sich parallel zur Längsachse des Behälters zu dem Behälter gerichtete bzw. axial gerichtete Verlängerungsringe 14 erstrecken. Die Verlängerungsringe 14 umschließen die Außenwand des Behälters und stellen zusammen mit zusätzlichen Dichtringen 5' einen druckdichten Abschluß für den Zellkulturraum 6 dar. Der obere Deckel 3 ist mit einer gemeinsamen Anschlußbohrung 8, 9 für die Zufuhr und den Rücklauf von Nährmedium versehen.

Fig. 6. zeigt eine Ausführungsform, ähnlich denen nach Fig. 5 Der wesentliche Unterschied besteht dabei darin, daß die Gewindeverbindung zwischen dem Behälter und den Deckeln 3 und 12 durch Innengewinde 4' in den Verlängerungsringen 14 gebildet ist, die mit Außengewinden 2' in dem Behälter zusammenwirken.

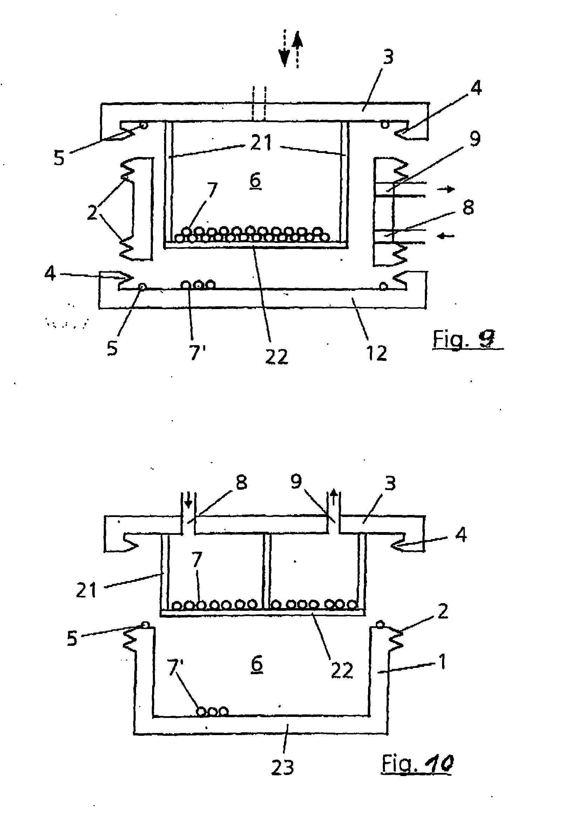

Fig. 7 zeigt eine Ausführungsform mit einem Behälter und einem oberen Deckel 3, ähnlich der Ausführungsform nach der Fig. 6, wobei anstelle einer gemeinsamen Anschlußbohrung 8, 9 für die Zufuhr und die Abfuhr von Nährmedium in der Umfangswand des Behälters eine Zulaufanschlußbohrung 8 und eine Rücklaufanschlußbohrung 9 angeordnet sind. Zusätzlich zeigt die Fig. 7 zwei seitliche Spannringe 15, die um den dosenförmigen Behälter und den Deckel 3 in Pfeilrichtung aufgeschoben werden, um die Einheit aus Behälter 1 und Deckel 3 durch eine nicht dargestellte Dreh- oder Rolleinrichtung um die Querachse in Pfeilrichtung 16 zu drehen oder zu rollen.

Fig. 8 zeigt eine erfindungsgemäße Ausführungsform. In diesem Fall ist in dem Inneren des Behälters ein eigener Zellkulturraum 6 gebildet. Anstelle eines Zellkulturraumes 6 kann auch eine Struktur vorgesehen sein, auf der die Zellen 7 gezüchtet werden. Der separate Zellkulturraum 6 oder die Struktur wird in diesem Falle über eine Druckbeaufschlagungseinrichtung 17 in Form einer Zylinder-/Kolbeneinheit mit Druck beaufschlagt.

In einem Kolbenraum 18 der Zylinder-/Kolbeneinheit 17 mündet die Zulaufanschlußbohrung 8, welche im Eingangsbereich durch ein Rückschlagventil 19 absperrbar ist. Durch einen Kolben 20 der Zylinder-/Kolbeneinheit 17 wird das über die Zulaufanschlußbohrung 8 eingebrachte Nährmedium unter Druck gesetzt, wobei sich dieser Druck bis in das Innere des Behälters 1 fortsetzt. Über eine Rücklaufanschlußbohrung 9 auf der dem Zulauf gegenüberliegenden Seite des Behälters erfolgt eine Abführung von Nährmedium. Wenn man den Innenraum des Behälters entsprechend unter einen Überdruck setzen möchte, der gegebenenfalls wechselnd sein kann, wird man in diesem Falle den Rücklauf von Nährmedium drosseln oder die Rücklaufanschlußbohrung 9 entsprechend absperren.

Anstelle einer Zufuhr von Nährmedium über die Zulaufanschlußbohrung 8 kann hierfür auch eine gesonderte Bohrung in einen der beiden Deckel 3 oder 12 oder in der Umfangswand des Behälters 1 vorgesehen sein. In diesem Falle kann man auch durch Gas, z.B. Luft, den Innenraum des Behälters 1 und damit den Zellkulturraum 6 entsprechend unter Druck setzen.

Die Fig. 9 zeigt eine Ausführungsform mit einem oberen Deckel 3 und einem unteren Deckel 12 und einem zylinderringförmigen Mittelteil eines Behälters. In diesem Falle ist der obere Deckel 3 mit einer Hängeeinrichtung in Form von mehreren über den Umfang verteilt angeordneten Stäben 21 versehen, die sich parallel zur Längsachse des Behälters 1 in das Innere des Behälters 1 erstrecken. Am unteren Ende der Stäbe 21 ist eine Plattform 22 befestigt, auf der die zu kultivierenden oder zu züchtenden Zellen 7 angeordnet werden. Die Zulaufanschlußbohrung 8 und die Rücklaufanschlußbohrung 9 können jeweils in der Umfangswand des Behälters angeordnet sein. Selbstverständlich ist jedoch auch eine Anordnung in einem der beiden Deckel 3 oder 12 möglich, wie dies gestrichelt angedeutet ist. Auch hier sind selbstverständlich auch getrennte Anschlußbohrungen für Zulauf und Ablauf möglich.

Der Vorteil der Ausführungsform mit der Hängeeinrichtung durch die Stäbe 21 besteht darin, daß sich auf diese Weise die Zellen 7 leichter in den Behälter 1 einsetzen und daraus entnehmen lassen.

Die Verbindung der Stäbe 21 mit der Plattform 22 kann im Bedarfsfalle auch lösbar sein. Eine Lösbarkeit kann z.B. durch eine Clipverbindung erfolgen, wodurch ebenfalls eine leichtere Handhabung für die Vorrichtung erreicht wird.

Fig. 10 zeigt eine Ausführungsform, die ähnlich der in der Fig. 9 dargestellten Ausführungsform ist. Wie ersichtlich, weist sie nur einen oberen Deckel 3 und einen Behälter mit einem Boden 23 auf, wie er auch bei den übrigen Ausführungsformen mit nur einem Deckel 3 vorhanden ist. Die Plattform 22 ist in diesem Falle ebenfalls über Stäbe 21 mit dem oberen Deckel 3 verbunden.

Ein weiterer Vorteil der Ausführungsformen nach den Fig. 9 und 10 besteht darin, daß auf dem Behälterboden 23 bzw. der Innenseite des Deckels 12 zusätzlich noch eine weitere Möglichkeit für eine Züchtung oder Kultivierung von Zellen 7 gegeben ist. Auf diese Weise wird ein Zweikammersystem für die Kultivierung von zwei Zellkulturen geschaffen.

Anstelle von Zulaufanschlußbohrungen 8 und Rücklaufanschlußbohrungen 9 für Nährmedium kann selbstverständlich auch Nährmedium dauerhaft in den Behälter eingebracht werden und die Zulaufanachlußbohrungen 8 und die Rücklaufanschlußbohrungen 9 können dann nur zur Sauerstoffversorgung dienen.

Alternativ dazu ist es auch möglich, jeweils gesonderte Anschlußbohrungen für Sauerstoff und Nährmedium vorzusehen.

Die Plattform 22 kann als feste Einheit ausgebildet sein. Alternativ dazu ist es auch möglich, hierfür eine Membrane, z.B. eine poröse Membrane, vorzusehen, die eine Durchströmung von Sauerstoff zuläßt.

## Patentansprüche

1. Vorrichtung zum Züchten oder Kultivieren von Zellen bestehend aus einem dosenartigen Behälter, der aus einem oberen Deckel (3) und einem unteren als Boden dienender Deckel (12) sowie einem zylinderförmigen Mittelteil (1) gebildet wird, welcher an beiden Stirnseiten druckdicht mit besagten Deckeln verbunden wird, wobei der Behälter mit einer Anschlussbohrung (8) zur Zufuhr und einer Anschlussbohrung (9) zur Abfuhr von Nährmedium versehen ist,
**dadurch gekennzeichnet, dass** besagte Deckel (3, 12) und der zylinderförmige Mittelteil (1) durch eine Gewindeverbindung (2, 4) miteinander verbunden sind, wobei beide Deckel (3, 12) jeweils mit einem Verlängerungsring (14) versehen sind, welche wenigstens teilweise das zylinderförmige Mitteilteil (1) außenseitig dichtend umschließen, und dass weiterhin an die Zulaufbohrung (8) eine für wechselnde Druckbelastung der Zellen (7) vorgesehene Druckbeaufschlagungseinrichtung, die als Zylinder/Kolbeneinheit (17) ausgebildet ist, angeschlossen ist, und der Behälter auf beiden Seiten mit einem Spannring (15) zur Einleitung von Roll- oder Drehbewegung für den Behälter versehen ist.

2. Vorrichtung nach Anspruch 1, **dadurch gekennzeichnet, dass** die Deckel (3, 12) ein Innengewinde und das zylinderförmige Mittelteil (1) ein Außengewinde besitzen.

3. Vorrichtung nach Anspruch 1 oder 2, **dadurch gekennzeichnet, dass** die Gewindeverbindung mit wenigstens einem Dichtring (5) versehen ist.

4. Vorrichtung nach Anspruch 1, **dadurch gekennzeichnet, dass** der Deckel (3) mit einer Hängeeinrichtung (21) versehen ist, an der eine Plattform (22) zur Aufnahme der Zellen (7) angeordnet ist.

5. Vorrichtung nach einem der Ansprüche 1-4, **dadurch gekennzeichnet, dass** die Zellen (7) in einem Gel (32) angeordnet sind.

6. Verwendung einer Vorrichtung gemäß der Ansprüche 1 - 5 für die Züchtung und Kultivierung von Zellen unter in vivo Bedingungen und Sterilität im Massenbetrieb in einem aktiven Prozess bei kontinuierlicher oder diskontinuierlicher Zuführung von Nährmedium und Aussetzen der sich bildenden Zellkulturen mit Druck.

## Claims

1. Apparatus for the culturing or cultivation of cells, consisting of a can-like container which is formed from an upper lid (3) and a lower lid (12) serving as base and a cylindrical central part (1), which is connected to said lids in a pressure-tight manner at both end faces, where the container is provided with a connection hole (8) for the supply of nutrient medium and a connection hole (9) for the discharge of nutrient medium,
**characterized in that** said lids (3, 12) and the cylindrical central part (1) are connected to one another by a threaded joint (2, 4), where the two lids (3, 12) are each provided with an extension ring (14) which at least partly surrounds the cylindrical central part (1) on the outside in a sealing manner, and **in that** furthermore a pressurization device, which is designed as cylinder/piston unit (17) and is intended for varying pressurization of the cells (7), is connected to the feed hole (8), and the container is provided on both sides with a clamp ring (15) for the initiation of rolling or rotating movement for the container.

2. Apparatus according to Claim 1, **characterized in that** the lids (3, 12) have an internal thread and the cylindrical central part (1) has an external thread.

3. Apparatus according to Claim 1 or 2, **characterized in that** the threaded joint is provided with at least one sealing ring (5).

4. Apparatus according to Claim 1, **characterized in that** the lid (3) is provided with a suspension device (21), on which a platform (22) for the accommodation of the cells (7) is arranged.

5. Apparatus according to one of Claims 1-4, **characterized in that** the cells (7) are arranged in a gel (32).

6. Use of an apparatus according to Claims 1-5 for the culturing or cultivation of cells under in-vivo conditions and sterility in mass operation in an active process with continuous or discontinuous supply of nutrient medium and pressurization of the cell cultures forming.

## Revendications

1. Appareil pour la culture ou la mise en culture de cellules, constitué par un conteneur en forme de boîte qui est formé à partir d'un couvercle supérieur (3) et d'un couvercle inférieur (12) jouant le rôle de base et à partir d'une partie centrale cylindrique (1), laquelle est connectée auxdits couvercles d'une manière étanche à la pression au niveau de deux faces d'extrémité, où le conteneur est muni d'un trou de connexion (8) pour l'application d'un milieu de nutriments et d'un trou de connexion (9) pour la décharge d'un milieu de nutriments,
**caractérisé en ce que** lesdits couvercles (3, 12) et la partie centrale cylindrique (1) sont connectés les uns aux autres par un joint fileté (2, 4), où les deux couvercles (3, 12) sont chacun munis d'une bague d'extension (14) qui entoure au moins partiellement la partie centrale cylindrique (1) sur l'extérieur d'une manière étanche, et **en ce qu'**en outre un dispositif de pressurisation, qui est conçu en tant qu'unité cylindre/piston (17) et qui est destiné à faire varier la pressurisation des cellules (7), est connecté au trou d'alimentation (8), et le conteneur est muni sur les deux côtés d'une bague de fixation (15) pour l'initiation d'un mouvement de roulement ou de rotation pour le conteneur.

2. Appareil selon la revendication 1, **caractérisé en ce que** les couvercles (3, 12) comportent un filet interne et la partie centrale cylindrique (1) comporte un filet externe.

3. Appareil selon la revendication 1 ou 2, **caractérisé en ce que** le joint fileté est muni d'au moins une bague d'étanchéité (5).

4. Appareil selon la revendication 1, **caractérisé en ce que** le couvercle (3) est muni d'un dispositif de suspension (21), sur lequel une plate-forme (22) pour le logement des cellules (7) est agencée.

5. Appareil selon l'une des revendications 1 à 4, **caractérisé en ce que** les cellules (7) sont agencées dans un gel (32).

6. Utilisation d'un appareil selon les revendications 1-5 pour la culture ou la mise en culture de cellules sous des conditions in vivo et sous un fonctionnement de stérilité en masse selon un processus actif avec une application continue ou discontinue d'un milieu de nutriments et une pressurisation de la formation des cultures de cellules.
